# EUROPEAN PATENT SPECIFICATION

(11) **EP 0 527 951 B1**
(45) Date of publication and mention of the grant of the patent: **13.11.1996**
(21) Application number: 91911536.0
(22) Date of filing: 07.05.1991
(51) Int. Cl.: A61M 25/00, A61M 39/00

(54) **MEDICAL INTRAVENOUS ADMINISTRATION LINE CONNECTOR**
VERBINDUNGSSTÜCK FÜR MEDIZINISCHE LEITUNG ZUR INTRAVENÖSEN VERSORGUNG
RACCORD POUR TUBE MEDICAL D'ADMINISTRATION PAR VOIE INTRAVEINEUSE

(30) Priority: 07.05.1990 US 520022
(43) Date of publication of application: 24.02.1993
(73) Proprietor: Winfield Industries Incorporated, San Diego, California 92121 (US)
(72) Inventor: RYAN, Dana, Wm., Franklin, TN 37604 (US)
(74) Representative: Austin, Hedley William
(86) International application number: US9103151
(87) International publication number: WO9116938

(56) References cited:
- EP-A- 0 319 764
- WO-A-91/07206
- US-A- 3 986 508
- US-A- 4 439 188
- US-A- 4 511 359
- US-A- 4 752 292
- US-A- 4 981 469

## Description

### BACKGROUND OF THE INVENTION

This invention relates to connectors for use with intravenous administration lines and systems in the medical field, and more particularly relates to a two part IV quick-connect/disconnect safety assembly that makes it more convenient to attach and detach a patient to and from an IV system.

Intravenous therapy has a long history of use in supplying patients with medicament, nourishment or fluids. One of the problems associated with intravenous therapy of ambulatory patients is that the patient cannot be easily disconnected from and reconnected to an intravenous administration line for short periods of time. To safely and easily disconnect the patient for even a short period of time requires the assistance of skilled medical personnel. The ambulatory patient is often required to be attached to the intravenous system and must wheel a stand holding the IV liquid supply wherever the patient goes. Being restricted in this manner can cause the patient to forgo activities of short duration that would be beneficial to the patient.

It is often necessary for hospital staff to move patients from location to location within the hospital in order to perform tests and certain medical treatments. It is not necessary or desirable to have the patient hooked up to the intravenous system during some of these activities. Removing a patient from an intravenous system and re-establishing the patient on the system takes a substantial amount of time even for a medical professional skilled in the techniques of intravenous therapy. With hospital costs rising dramatically there is considerable advantage in a connector device that allows the medical staff to be able to quickly and safely connect and disconnect a patient from an intravenous therapy administration line while expending a minimum amount of time in doing so.

The prior art has addressed some of the above stated problems. For example, patent 4,511,359 issued to Vaillancourt describes a three-part sterile dialysis connection device for home use. The three parts are a male connector which terminates in a catheter tube; a female connector with a hollow needle secured in place and terminating in a flexible tubing; and a molded septum assembly. Vaillancourt places the molded septum assembly in the receiver end of the female connector. The female connector is then slid over the male connector, thereby pushing the septum assembly into place between the male and female connectors, and into friction fit with the male connector, and also causing the sharpened needle in the female connector to pierce the molded septum assembly. The hollow needle provides a path for fluid flow between the two connector parts. When the male and female connectors are separated, the needle is removed from the self-sealing septum, and the septum assembly remains with and covering the male connector because of its friction fit therewith.

Another three-part home dialysis connection device is described in patent 4,810,241 issued to Rogers which provides a sterile connection by mechanical and chemical means. The three parts include two connectors, one attached to an influent tube and the other to the catheter tube, and a cylindrical shaped tube in which there is highly absorbent material saturated with antiseptic. The two connectors in turn connect one to each end of the cylinder. As the end connectors are introduced into the central cylinder connector, they are sterilized by the antiseptic in the cylinder and remain in antiseptic contact during the entire time they are being used for dialysis. A sterile environment is maintained on the catheter tubing side of the IV system only for so long as the catheter side tubing is in the connector cylinder. Care must be taken not to let the disinfectant in the cylinder dry out or evaporate.

A somewhat different solution to the problem was taken by patent 4559043 issued to Whitehouse et al which provides a four-piece assembly including a distal connector, a proximate connector, a septum fitting between and held in place by the distal and proximate connectors, and an adapter with a through bore able to accept a hollow needle, the adapter being used in conjunction with the proximate connector. A hollow needle attached to a standard luer extension T which connects to the adapter is pushed through the adapter needle bore and pierces the septum which is held between the proximate and distal connectors, thereby establishing fluid flow. When the hollow needle attached to the luer T is removed, the septum is sealed, but the needle is exposed, presenting a needlestick hazard.

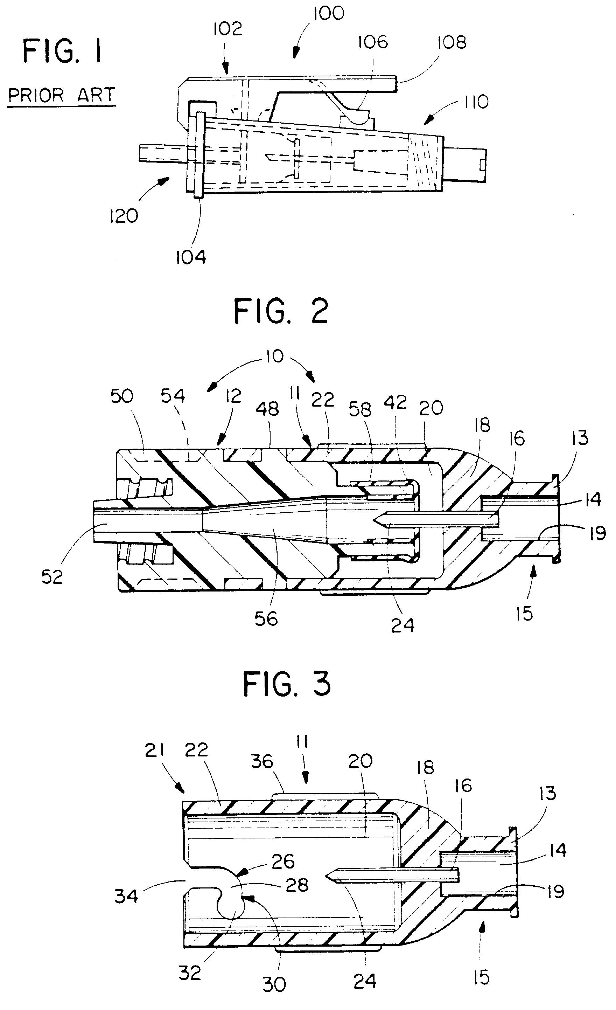

The prior art of the Figure 1 shows a connector 100 manufactured by ICU Medical Inc., Irvine, California and illustrates the extreme complexity some IV line connectors have embodied in an attempt to solve the problems in the art. The prior art connector 100 has a female portion 110 and a male portion 120. A spring loaded thumb lever 102 which protrudes from the side of the female connector 110 is provided to clamp a rear flange of the male connector 120 against the end surface 104 of the female connector 110. The plastic spring 106 shown in Figure 1 provides the spring action which holds the lever 102 in the locked position. The connector is complex and difficult to manufacture, expensive to make, and potentially hazardous due to the high probability of the lever 102 becoming caught on bed rails, medical equipment, or tubing at a patient's bedside.

United States patent 4752292 discloses in Figures 23 and 24 a fluid line connector having a relatively greater diameter female cap member connector arranged to receive a lesser diameter male connector. The female cap member carries a needle arranged to pierce a seal on an end of the male connector when connected. Bayonet slits and projections co-operate to guide and secure coupling connection of the female cap member connector and male connector.

In particular this fluid line assembly comprises a female connector having first and second hollow portions, a dividing wall separating said first and second hollow portions, and a hollow needle having a first sharp end, said first hollow portion having a first substantially cylindrical wall forming a receiving chamber with a first substantially open end, said first substantially cylindrical wall having at least one bayonet cutout therethrough, said second hollow portion having a second substantially cylindrical wall with an inside surface for mating with an influent supply line, and an outside surface of reduced outer diameter relative to the outer diameter of most of said first substantially cylindrical wall, and said hollow needle forming a passageway through said dividing wall, with said first sharp end of said hollow needle extending into said receiving chamber; a male connector with a hollow first end cylindrical portion having a first opening, a hollow middle body cylindrical portion, a hollow second end cylindrical portion for mating with an effluent supply line extending therefrom, and a resilient septum, said hollow first end cylindrical portion being fitted with said resilient septum at said first opening, and said hollow first end cylindrical portion and said hollow middle body cylindrical portion being of reduced outer diameters relative to the inner diameter of said receiving chamber of said female connector, said hollow middle body cylindrical portion having at least one outwardly extending bayonet knob, the outer diameter of said extending bayonet knob being of substantially the identical outer diameter of the outer diameter of said receiving chamber, and wherein said bayonet cutout includes a locking section, and said bayonet cutout and said outwardly extending knobs are dimensioned such that said outwardly extending knobs are received in said bayonet cutout and can lock in said locking section, and the axial distance between the locking section of said bayonet cutout and the sharp end of said needle along a longitudinal axis of said female connector is smaller than the axial distance between said septum and said outwardly extending knobs along a longitudinal axis of said male connector such that when said female connector and male connector are mated, said sharp end of said needle pierces said septum.

United States patent 3986508 discloses a fluid line connector for use in processing blood which comprises relatively greater diameter female body element arranged to engage with a relatively narrower male element to effect puncturing of male and female septa to provide a fluid passageway through the connector.

While the devices of the prior art may be effective for their particular purposes, the requirement for a simple, low-cost, quick-connect/disconnect safety assembly is not filled. The prior art does not show a device which has all of the virtues in a single device of being simple and inexpensive to manufacture, providing standard means such as luer fittings for attachment to other devices, and providing means for shielding the needle after use to prevent accidental needlesticks.

It is therefore as the object of the invention to provide a simple, low cost, IV quick-connect/disconnect safety assembly.

Another object is to provide an IV quick-connect/disconnect assembly that is streamlined and has no projections or protruding parts which might catch or snag.

A further object of the invention is to provide an IV quick-connect/disconnect assembly designed so that the needle piercing the septum of the male connector is contained in a shielded area at all times, thereby substantially minimizing the probability of accidental needlesticks.

Another object of the invention is to provide an IV quick-connect/disconnect assembly that will reduce the average time it takes medical personnel to establish the IV therapy system servicing a patient.

Yet another object of the invention is to provide an IV quick-connect/disconnect assembly that has means at both ends that permit attachment to any type of standard administration lines, extension sets, winged needles, catheters, or other IV medical devices using standard medical attachment means such as luer-locks or luer-slip fittings.

An even further object is to use the female portion of the quick-connect/disconnect assembly in conjunction with a syringe device to provide a safety syringe which may be used for injection into an IV administration line or PRN device.

In accordance with the objects stated above an IV quick-connect/disconnect assembly is provided and generally comprises an assembly in accordance with the appended claims. When mated, the surfaces of the male and female connectors form a smooth streamlined surface with no projections that can catch or snag other medical equipment.

In using the male and female connectors, an IV administration line with a male luer-lock or luer-slip is inserted into the female luer side of the female connector, while an IV winged needle or catheter device that is connected directly to an extension line or the like which terminates in a female luer-lock or luer-slip is connected with the male luer-lock or luer-slip of the male connector. The male and female connectors are mated by sliding the reduced diameter second end of the male connector into the receiving cylinder of the female connector, with the cutouts of the female connector serving as a track for the extended knobs of the male connector. As the male connector is slid forward, the needle in the female connector pierces the resilient septum which permits the flow of liquid through the septum via the hollow needle. The connectors are locked into place by bringing the male connector as far forward as possible, and then rotating the male connector such that the extended knobs move past the restriction in the cutout and click (lock) into place. Quick release is obtained by rotating the male connector in the opposition direction and pulling the male connector straight out relative to the female connector. When the male and female connectors are separated, the needle in the female connector is withdrawn from the self-sealing resilient septum attached to the reduced diameter second end of the male connector.

The male connector of the IV quick-connect/disconnect safety assembly may be fabricated as the termination of the branch of a Y-site on an extension set. This makes it possible to add a second source of IV fluid via a bag, bottle, or syringe. Where a piggyback or secondary extension set is utilized, a female connector for mating with the male Y terminator is provided, as summarized above. However, where a syringe is utilized the syringe is preferably coupled with its male luer-lock or luer-slip to the female luer of the above-summarized female connector. That assembly is then connected to the male connector which completes the assembly and establishes the flow path in the IV administration system. When the syringe is removed after injection, the female connector stays attached to the syringe and reduces the probability of accidental needle-sticks.

Additional objects and advantages of the invention will become apparent to those skilled in the art upon reference to the detailed description in conjunction with the accompanying drawings.

### BRIEF DESCRIPTION OF THE DRAWINGS

Fig. 1 is a perspective of a two piece prior art connector utilizing a spring loaded lever for locking together a male and a female connector.

Fig. 2 is a cross section through the IV quick-connect/disconnect safety assembly invention.

Fig. 3 is a cross section through the female connector of the IV quick-connect/disconnect safety assembly invention.

Fig. 4 is a cross section through the male connector of the IV quick-connect/disconnect safety assembly invention.

Fig. 5 is a cross section through the female connector of the IV quick-connect/disconnect safety assembly invention showing its use with a syringe having a luer-lock on its distal end.

Fig. 6a - 6c illustrate the location and relationship of the IV connector assembly of the invention relative to the other components that make up possible IV administration systems.

### DETAILED DESCRIPTION OF THE INVENTION

As seen in Fig. 2, the IV quick-connect/disconnect safety assembly 10 of the invention comprises a female connector 11 and male connector 12. The female connector 11, as shown in Fig. 3, has a reduced end 15 and an enlarged end 21 separated by a dividing wall 18. The reduced end 15 forms a female luer lock with a female luer 19 and a tab 13. The enlarged end 21 is defined by a cylindrical wall 22 which surrounds a cylindrical receiving area 20.

A hollow needle 16 is insert molded or bonded in the dividing wall 18 so that the pointed end of the needle 24 is located in the receiving cylinder, the hollow needle forming a passageway between the tapered bore 14 of the female luer lock and the receiving cylinder. The receiving cylinder wall 22 has one or more bayonet type cutouts 28 having a first section which is parallel to the long axis of the female connector and a second section which is perpendicular to and a continuation of the first section in the perpendicular direction. Entrance to the cutout 34 is at the open end of the receiving cylinder, and the cutout has a restriction 30 which reduces the entry way into the circular area 32. The female connector is molded from plastic or other acceptable materials and has a plurality of integral tapered, smooth, plastic ribs 36 on the exterior surface of the cylindrical wall 22 running in the direction of the longitudinal axis of the female connector which serve the purpose of providing finger grips.

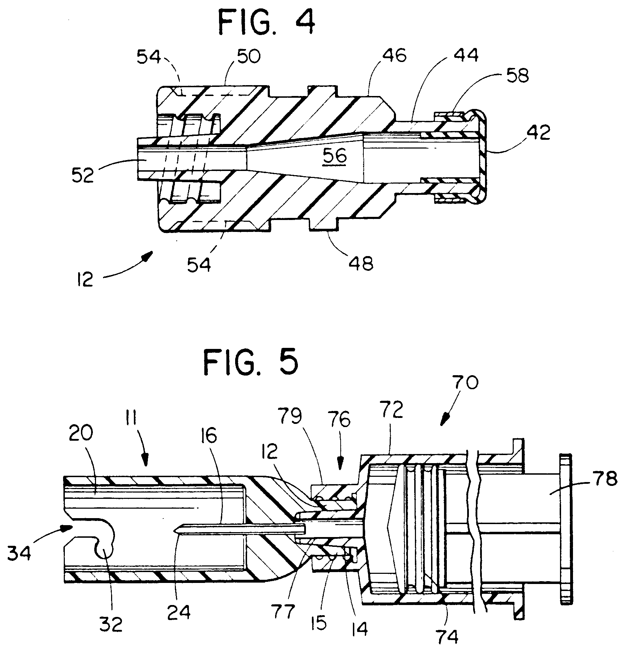

The male connector 12, shown in Fig. 4, has a male luer lock 52 on a first end 50, a middle portion 46 of slightly reduced outer diameter with one or more outwardly extending rounded knobs 48, and a second end 44 of further reduced outer diameter. First end 50 is preferably ribbed by cutting into the exterior cylindrical surface as shown by dashed lines 54 to form a finger grip. Second end 44 is covered by a resilient septum 42 fitted to and preferably covering at least a portion of both the interior and exterior wall surface of second end 44. The resilient septum is held in place by a thin plastic shrink band 58 or other acceptable means which surrounds the outer surface of the septum on the second end 44. The first end 50, the middle portion 46, and the second end 44 of the male connector 12 encompass a bore 56 which preferably enlarges as it proceeds from the first end 50 towards the second end 44 of the male connector 12.

In connecting the male and female connectors to make up the invention assembly as shown in Fig. 2, the male connector is positioned so that the reduced diameter second end 44 of the male connector is introduced into the receiving chamber 20 of the female connector 11 with the rounded knobs 48 of the male connector being in position at the openings 34 of the bayonet cutouts 28 (see Fig. 3) in the female connector. The male connector is pushed forward as far as it will go until the knobs come in contact with the cutout surface shown as 26. The male connector is then rotated so that the knobs go past the restriction 30 in the radius wall and come to rest in the cutout pockets 32 shown in Fig 3. The cutout pockets 32 in conjunction with the restriction 30 apply sufficient force to the knobs to securely hold the assembly together. When the male and female connectors are locked together, a distinct audible click is heard signaling to the person assembling the connectors that the connection is secure.

When the reduced diameter 44 of the male connector 12 is pushed forward as described above, the needle 16 of the female connector 11 pierces the septum 42 and permits fluid to flow from chamber 14 shown in Figs. 2 and 3 through the needle 16 and into bore 56. When the male and female connectors are mated, the knobs 48 of the male connector are substantially flush with the cylindrical wall 22 of the receiving cylinder which is part of the female connector. As a result, the outside surfaces of the male and female connector when joined present a smooth surface; there are no projections outward from the body of the assembled connector.

When the male and female connectors are to be disconnected, the male connector 12 is rotated so that the knobs 48 move past the restriction 30 in the bayonet cutouts in cylindrical wall 22 of the female connector 11. Then the male connector 12 is pulled straight back so that the knobs pass down the longitudinal part of the cutouts to the entry way 34. As the male connector is removed, the needle is withdrawn from the resilient self-sealing septum 42 which immediately seals and cuts off fluid flow. The self-sealing septum 42 also keeps out bacteria, dirt, dust and other contaminates from the patient side of the IV administration system. When disconnected, the needle 16 in the female connector 11 is shielded by the receiving cylinder wall 22 so that inadvertent needlesticks are reduced to a minimum. This is an important safety feature.

In the preferred embodiment, the middle portion 46 of the male connector 12 is sized to loosely contact the receiving cylinder walls 22 of the female connector when mating, thereby serving the added function of centering the male connector so that the needle point 24 is centered when it pierces the septum 42. The line connector device may be reused without negative effect on the functioning of the device.

Use of the female connector 11 in conjunction with a syringe 70 for introducing medicament or other fluid into an IV system is illustrated by Fig. 5. As previously described, the female connector 11 has a female luer-lock or luer-slip on its reduced end 15. A syringe 70 with a cylindrical barrel formed by wall 72 has a reduced end 76 forming either a male luer-lock or luer-slip (the luer-lock being shown). The male luer portion 77 of the luer-lock or luer-slip is inserted into the tapered bore 14 formed by the female luer-lock or luer-slip of the female connector 11 by screwing or pushing the two together. When the female connector 11 and the syringe 70 are assembled in this manner, fluid in the syringe 70 may be expelled from the syringe by plunger 78, thereby forcing the fluid through the hollow needle 16 and into the IV administration line. After injection, the syringe 70 with the female connector 11 attached thereto is removed from the resilient septum which then seals. The needle 16, however, which could have been infected in the injection process due to migratory bacteria or blood contamination, is not exposed as it is shielded by the receiving cylinder wall 22 of the female connector 11. The use of the syringe 70 and female connector 11 combination is thus attractive because accidental needlesticks are substantially reduced.

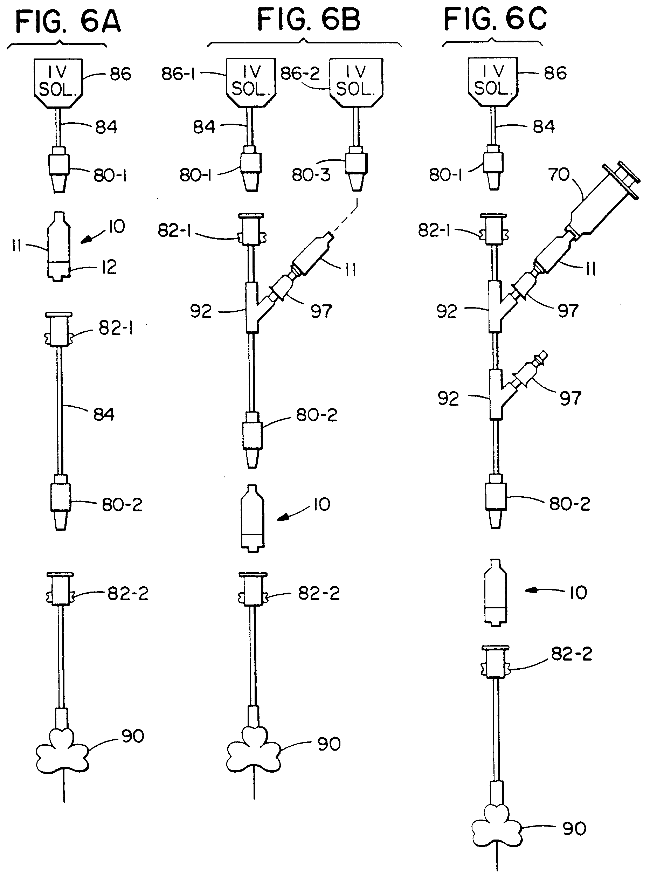

Fig. 6a shows the relationship of the assembled connector 10 in a typical IV administration system. On the medication side of the quick-connect/disconnect assembly 10, the IV solution 86 is connected via tubing 84 which has a male luer 80-1 on its end. The male luer is inserted into a female luer-lock of the female connector 11 of the IV quick-connect/disconnect safety assembly 10 of the invention. On the patient side of the quick-connect/disconnect assembly 10, an extension tube with two ends has a first end with a female luer 82-1 which is inserted into a male luer-lock 50 of the IV quick-connect/disconnect safety assembly male connector 12. A second end of the extension tube has a male luer 80-2 attached to it which in turn mates with a female luer 82-2 on the end of a catheter, winged needle, or other IV device 90. To permit fluid flow of medication to the patient, the female connector 11 and the male connector 12 which make up the complete quick-connect/disconnect assembly 10 are mated.

Fig. 6b shows a similar type IV administration system to that of Fig. 6a except that a Y-site connector 92 is placed in conjunction with an extension line group (female luer 82-1, line 84, and male luer 80-2) between the IV solution 86-1 and the assembled quick-connect/disconnect device 10 of the invention. The Y-site connector 92 allows other medicaments and fluids to be administered through the septum fitted male connection (PRN) 97 shown as the termination of the branch of the Y-site connector 92, such as by utilizing the female connector 11 of the invention in conjunction with male luer 80-3 and bag or bottle 86-2. Regardless of whether the PRN device 97 shown in Fig. 6b is the male connector 12 of the IV quick-connect/disconnect safety assembly of the invention, or a connector of another manufacturer, the female connector 11 and syringe 70 assembly described above with reference to Figure 5 can be used as a safety combination for injecting medication into the IV administration line (as more particularly seen in Fig. 6c). In addition, if the PRN device 97 is indeed the male connector 12 of the quick-connect/disconnect assembly 10 of the invention, a female connector 11 attached to another fluid source (not shown) could be utilized in the manner previously described to establish a second quick-connect/disconnect fluid path.

Fig. 6c shows that it is possible to introduce more than one Y-site connector 92 at a time into the IV system. In fact, it will be appreciated by those skilled in the art that the components shown in Figures 6a-6c can be mixed and matched in several ways at the convenience of the practitioner. In addition, groups of components can be manufactured or assembled as a group to perform standard functions; e.g. female luer 82, line 84 and male luer 80 forming an extension line; bag or bottle 86, line 84 and male luer 80 forming a fluid source; catheter or winged needle element 90, line 84, and female luer 82 forming a catheter, winged needle, or other IV device connection group.

## Claims

1. A fluid line connection assembly for permitting the coupling and uncoupling of an influent supply line (84) terminating in a first male luer (80) and an effluent supply line starting with a first female luer (82), the assembly comprising:
a) a female connector (11) having first (21) and second (15) hollow portions, a dividing wall (18) separating said first and second hollow portions, and a hollow needle (16) having a first sharp end (24),
said first hollow portion (21) having a first substantially cylindrical wall (22) forming a receiving chamber with a first substantially open end, said first substantially cylindrical wall having at least one bayonet cutout (28) therethrough,
said second hollow portion (15) having a second substantially cylindrical wall with an inside surface forming a second female luer (19) for mating with said first male luer (80) of said influent supply line, and an outside surface of reduced outer diameter relative to the outer diameter of most of said first substantially cylindrical wall (22), and
said hollow needle (16) forming a passageway through said dividing wall (18) and connecting said receiving chamber and said second female luer (19), with said first sharp end (24) of said hollow needle (16) extending into said receiving chamber;
b) a male connector (12) with a hollow first end cylindrical portion (44) having a first opening, a hollow middle body cylindrical portion (46), a hollow second end cylindrical portion (50) with a second male luer (52) for mating with said first female luer (82) of said effluent supply line extending therefrom, and a resilient septum (42),
said hollow first end cylindrical portion (44) being fitted with said resilient septum (42) at said first opening, and said hollow first end cylindrical portion (44) and said hollow middle body cylindrical portion (46) being of reduced outer diameters relative to the inner diameter of said receiving chamber of said female connector (11),
said hollow middle body cylindrical portion (46) having at least one outwardly extending bayonet knob (48), the outer diameter of said extending bayonet knob being of substantially the identical outer diameter of the outer diameter of said receiving chamber, and
the outer diameter of said hollow second end cylindrical portion (50) being substantially identical to the outer diameter of said receiving chamber, wherein
said bayonet cutout (26) includes a locking section (30), and said bayonet cutout and said outwardly extending knobs (48) are dimensioned such that said outwardly extending knobs are received in said bayonet cutout and can lock in said locking section, and
the axial distance between the locking section (30) of said bayonet cutout (26) and the sharp end (24) of said needle (16) along a longitudinal axis of said female connector (11) is smaller than the axial distance between said septum (42) and said outwardly extending knobs (48) along a longitudinal axis of said male connector (12) such that when said said female connector and male connector are mated, said sharp end (24) of said needle (16) pierces said septum (42), and
when said female connector (11) and male connector (12) are mated, a substantially smooth, substantially continuous outer cylindrical surface is presented comprised of said outer surface of said hollow second end cylindrical portion (50) of said male connector and said first substantially cylindrical wall (22) of said female connector, with said outwardly extending knobs (48) of said male connector in said bayonet cutout (28) of said female connector.

2. A fluid line connection assembly according to claim 1, wherein:
said sharp end (24) of said needle (16) is recessed in said receiving chamber relative to said first substantially open end of said receiving chamber so as to reduce needlestick injuries.

3. A fluid line connection assembly according to claim 2, wherein said dividing wall (18) of said female connector (11) has a smooth outer surface which tapers from the outer diameter of said receiving chamber to the outer diameter of said second hollow portion (15) of said female connector.

4. A fluid line connection assembly according to claim 3, wherein said second female luer (19) of said female connector (11) comprises a female luer-lock.

5. A fluid line connection assembly according to claim 3 or 4, wherein said second male luer (52) of said male connector (12) comprises a male luer-lock.

6. A fluid line connector assembly according to claim 5, wherein said male connector (12) and said female connector (11) are moulded from plastics, said hollow second end cylindrical portion (50) of said male connector is moulded with indentations in its outer surface as finger grip means for sale male connector, and said first substantially cylindrical wall (22) of said female connector is moulded with tapered ribs (36) on its outer surface as finger grip means for said female connector.

7. A fluid line connection assembly according to any of claims 1 to 6, further comprising a plastic shrink band (58) for holding said resilient septum (42) in place in and around said first opening of said male connector (12).

8. A fluid line connection assembly according to any of claims 1 to 7, wherein said male connector (12) and female connector (11) are moulded from plastics.

9. A fluid line connector assembly according to claim 8, wherein said hollow second end cylindrical portion (50) of said male connector (12) is moulded with indentations in its outer surface as finger grip means for said male connector; and said first substantially cylindrical wall (22) of said female connector (11) is moulded with tapered ribs (36) on its outer surface as finger grip means for said female connector.

10. A fluid line connector assembly according to claim 8 or 9, wherein said hollow needle (16) with said sharp first end (24) is of plastics and is formed simultaneously with said female connector (11) in a moulding process.

## Patentansprüche

1. Verbindungsanordnung für eine Flüssigkeitsleitung, die das Kuppeln und Entkuppeln einer Zufluß-Versorgungsleitung (84), die in einem ersten männlichen Luer-Verbinder (80) endet und einer Abfluß-Versorgungsleitung, die mit einem ersten weiblichen Luer-Verbinder (82) beginnt, erlaubt, wobei die Anordnung umfaßt:
a) ein weibliches Verbindungsstück (11) mit einem ersten (21) und einem zweiten (15) hohlen Teil, einer Trennwand (18), die das erste und zweite Teil voneinander trennt, und einer hohlen Nadel (16) mit einem ersten scharfen Ende (24),
wobei der erste hohle Teil (21) eine erste im wesentlichen zylindrische Wand (22) aufweist, die eine Aufnahmekammer mit einem ersten im wesentlichen offenen Ende bildet, wobei die erste im wesentlichen zylindrische Wand wenigstens einen Bajonett-Ausschnitt (28) besitzt,
wobei der zweite hohle Teil (15) eine zweite im wesentlichen zylindrische Wand mit einer Innenfläche, die einen zweiten weiblichen Luer-Verbinder (19) bildet, der zu dem ersten männlichen Luer-Verbinder (80) der Zufluß-Versorgungsleitung paßt, und einer Außenfläche mit einem verminderten Außendurchmesser relativ zu dem Außendurchmesser des größten Teils der ersten im wesentlichen zylindrischen Wand (22) aufweist, und
wobei die hohle Nadel (16) einen Durchlaß durch die Trennwand (18) bildet und die Aufnahmekammer und den zweiten weiblichen Luer-Verbinder (19) verbindet, wobei das erste scharfe Ende (24) der hohlen Nadel (16) sich in die Aufnahmekammer erstreckt;
b) ein männliches Verbindungsstück (12) mit einem hohlen zylindrischen Teil (44) am ersten Ende mit einer Öffnung, einem mittleren hohlen zylindrischen Rumpfteil (46), einem hohlen zylindrischen Teil (50) am zweiten Ende mit einem zweiten männlichen Luer-Verbinder (52), der zu dem ersten weiblichen Luer-Verbinder (82) der von diesem ausgehenden Abfluß-Versorgungsleitung paßt, und einer nachgiebigen Trennwand (42),
wobei der hohle zylindrische Teil (44) am ersten Ende mit der nachgiebigen Trennwand (42) an der ersten Öffnung ausgestattet ist, und wobei der hohle zylindrische Teil (44) am ersten Ende und der mittlere hohle zylindrische Rumpfteil (46) verminderte Außendurchmesser relativ zu dem Innendurchmesser der Aufnahmekammer des weiblichen Verbindungsstücks (11) haben,
wobei der mittlere hohle zylindrische Rumpfteil (46) wenigstens einen sich nach außen erstreckenden Bajonett-Knopf (48) aufweist, wobei der sich nach außen erstreckende Bajonett-Knopf im wesentlichen einen identischen Außendurchmesser hat wie der Außendurchmesser der Aufnahmekammer, wobei
der Bajonett-Ausschnitt (26) einen Verriegelungsabschnitt (30) enthält und der Bajonett-Ausschnitt und die sich nach außen erstreckenden Knöpfe (48) so bemssen sind, daß die sich nach außen erstreckenden Knöpfe von dem Bajonett-Ausschnitt aufgenommen werden und in dem Verriegelungsabschnitt verriegelt werden können; und
wobei der axiale Abstand zwischen dem Verriegelungsabschnitt (30) des Bajonett-Ausschnitts (26) und das scharfe Ende (24) der Nadel (16) entlang einer Längsachse des weiblichen Verbindungsstücks (11) kleiner als der axiale Abstand zwischen der Trennwand (42) und den sich nach außen erstreckenden Knöpfen (48) entlang einer Längsachse des männlichen Verbindungsstücks (12) ist, so daß beim Zusammenstecken des weiblichen und des männlichen Verbindungsstücks das scharfe Ende (24) der Nadel (16) die Trennwand (42) durchsticht; und
wobei nach dem Zusammenstecken des weiblichen Verbindungsteils (11) und des männlichen Verbindungsstücks (12) eine glatte im wesentlichen kontinuierliche zylindrische Außenfläche dargeboten wird, die aus der Außenfläche des hohlzylindrischen Teils (50) am zweiten Ende des männlichen Verbindungsstücks und der ersten im wesentlichen zylindrischen Wand (22) des weiblichen Verbindungsstücks besteht, wobei die sich nach außen erstreckenden Knöpfe (48) des männlichen Verbindungsstücks sich in dem Bajonett-Ausschnitt (28) des weiblichen Verbindungsstücks befinden.

2. Verbindungsanordnung für eine Flüssigkeitsleitung nach Anspruch 1, bei der das scharfe Ende (24) der Nadel vertieft in der Aufnahmekammer relativ zu dem ersten im wesenlichen offenen Ende der Aufnahmekammer angeordnet ist, um so Nadelstichverletzungen zu vermindern.

3. Verbindungsanordnung für eine Flüssigkeitsleitung nach Anspruch 2, bei der die Trennwand (18) des weiblichen Verbindungsstücks (11) eine glatte Außenfläche hat, die sich vom Außendurchmesser der Aufnahmekammer zum Außendurchmesser des zweiten hohlen Teils (15) des weiblichen Verbindungsstücks verjüngt.

4. Verbindungsanordnung für eine Flüssigkeitsleitung nach Anspruch 3, bei der der zweite weibliche Luer-Verbinder (19) des weiblichen Verbindungsstücks (11) eine weibliche Luer-Verriegelung ist.

5. Verbindungsanordnung für eine Flüssigkeitsleitung nach Anspruch 3 oder 4, bei der der zweite männliche Luer-Verbinder (52) des männlichen Anschlußstückes (12) eine männliche Luer-Verriegelung ist.

6. Verbindungsanordnung für eine Flüssigkeitsleitung nach Anspruch 5, bei der das männliche Verbindungsstück (12) und das weibliche Verbindungsstück (11) aus Kunststoff geformt sind, wobei der hohlzylindrische Teil (50) am zweiten Ende des männlichen Verbindungsstücks an seiner Außenseite mit Vertiefungen als Fingergreifmittel für das männliche Verbindungsstück versehen ist, und wobei die erste im wesentlichen zylindrische Wand (22) des weiblichen Verbindungsstücks mit konischen Rippen (36) an ihrer Außenfläche als Fingergreifmittel für das weibliche Verbindungsstück versehen ist.

7. Verbindungsanordnung für eine Flüssigkeitsleitung nach einem der Ansprüche 1 bis 6, umfassend ein Kunststoffschrumpfband (58), um die nachgiebige Trennwand (42) an Ort und Stelle vor der ersten Öffnung des männlichen verbindungsstücks (12) zu halten.

8. Verbindungsanordnung für eine Flüssigkeitsleitung nach einem der Ansprüche 1 bis 7, bei der das männliche Verbindungsstück (12) und das weibliche Verbindungsstück (11) aus Kunststoff geformt sind.

9. Verbindungsanordnung für eine Flüssigkeitsleitung, bei der der hohlzylindrische Teil (50) am zweiten Ende des männlichen Verbindungsstücks (12) mit Vertiefungen an seiner Außenfläche als Fingergreifmittel für das männliche Verbindungsstück versehen ist; und wobei an der ersten im wesentlichen zylindrischen Wand (22) des weiblichen Verbindungsstücks (11) konische Rippen (36) an ihrer Außenfläche als Fingergreifmittel für das weibliche Verbindungsstück angeformt sind.

10. Verbindungsanordnung für eine Flüssigkeitsleitung nach Anspruch 8 oder 9, bei der die hohle Nadel (16) mit dem scharfen ersten Ende (24) aus Kunststoff besteht und gleichzeitig mit dem weiblichen Verbindungsstück (11) in einem Formprozeß gebildet wird.

## Revendications

1. Ensemble de raccord d'une conduite de fluide destiné à permettre l'accouplement et le désaccouplement entre une conduite d'alimentation entrant (84), se terminant dans un premier embout mâle (80), et une conduite d'alimentation sortant partant d'un premier embout femelle (82), l'ensemble comprenant:
a) un raccord femelle (11) ayant des première (21) et seconde (15) parties creuses, une paroi séparatrice (18) séparant lesdites première et seconde parties l'une de l'autre, et une aiguille creuse (16) ayant une première extrémité coupante (24),
ladite première partie (21) comportant une première paroi essentiellement cylindrique (22) formant une chambre réceptrice ayant une première extrémité essentiellement ouverte, ladite première paroi essentiellement cylindrique comportant au moins une encoche à baïonnette (28) ménagée dans la paroi,
ladite seconde partie creuse (15) comportant une seconde paroi essentiellement cylindrique dont la surface intérieure forme un deuxième embout femelle (19) destiné à s'adapter sur ledit premier embout mâle (80) de ladite conduite d'alimentation entrant, et dont la surface extérieure présente un diamètre extérieur réduit par rapport au diamètre extérieur de la plupart de ladite première paroi essentiellement cylindrique (22), et
ladite aiguille creuse (16) formant un passage à travers ladite paroi séparatrice (18) et reliant ladite chambre réceptrice audit second embout femelle (19), ladite première extrémité coupante (24) de ladite aiguille creuse (16) s'étendant a l'intérieur de ladite chambre réceptrice;
b) un raccord mâle (12) comportant une première partie terminale cylindrique (44) ayant une première ouverture, une partie de corps central creux cylindrique (46), un seconde partie terminale cylindrique (50) comportant un deuxième embout mâle (52) destiné a s'adapter dans ledit premier embout femelle (82) de ladite conduite d'alimentation sortant s'étendant à partir de cette conduite, et un opercule élastique (42),
ladite première partie terminale cylindrique (44) étant équipée dudit opercule élastique (42) au niveau de ladite première ouverture, et ladite première partie terminale cylindrique (44) et ladite partie de corps central creux cylindrique (46) présentant des diamètres extérieurs réduits par rapport au diamètre intérieur de ladite chambre réceptrice dudit raccord femelle (11),
ladite partie de corps central creux cylindrique (46) comprenant au moins un bouton a baïonnette (48) s'entendant vers l'extérieur, le diamètre extérieur dudit bouton à baïonnette étant essentiellement identique au diamètre extérieur de ladite chambre réceptrice, et
le diamètre extérieur de ladite seconde partie terminale cylindrique (50) étant essentiellement identique au diamètre extérieur de ladite chambre réceptrice;
dans lequel
ladite encoche à baïonnette (26) comporte un partie de verrouillage (30) et ladite encoche à baïonnette et lesdits boutons (48) s'étendant vers l'extérieur sont dimensionnés de manière telle que les boutons s'étendant vers l'extérieur sont reçus dans ladite encoche à baïonnette et peuvent se verrouiller dans ladite section de verrouillage, et
la distance axiale entre la section de verrouillage (30) de ladite encoche à baïonnette (26) et l'extrémité coupante (24) de ladite aiguille (16) le long d'un axe longitudinal dudit raccord femelle (11) est inférieure à la distance axiale comprise entre ledit opercule (42) et lesdits boutons (48) s'étendant vers l'extérieur, le long d'un axe longitudinal dudit raccord mâle (12), de manière telle que, lorsque ledit raccord femelle et ledit raccord mâle sont emboîtés l'un dans l'autre, ladite extrémité coupante (24) de ladite aiguille (16) perce ledit opercule (42), et
lorsque ledit raccord femelle (11) et ledit raccord mâle (12) sont emboîtes l'un dans l'autre, une surface cylindrique extérieure essentiellement continue lisse est produite, qui se compose de ladite surface extérieure de ladite seconde partie terminale cylindrique (50) dudit raccord mâle, et de ladite première paroi essentiellement cylindrique (22) dudit raccord femelle, lesdits boutons (48) dudit raccord mâle, s'étendant vers l'extérieur, s'engageant dans ladite encoche à baïonnette (28) dudit raccord femelle.

2. Ensemble de raccord d'une conduite de fluide selon la revendication 1, dans lequel:
ladite extrémité coupante (24) de ladite aiguille (16) est en retrait par rapport à ladite première extrémité essentiellement ouverte de ladite chambre réceptrice, à l'intérieur de ladite chambre réceptrice, de manière à réduire les risques de blessures par l'aiguille.

3. Ensemble de raccord d'une conduite de fluide selon le revendication 2, dans lequel ladite paroi séparatrice (18) dudit raccord femelle (11) présente une surface extérieure lisse qui se rétrécit de façon conique du diamètre extérieur de ladite chambre réceptrice jusqu'au diamètre extérieur de ladite seconde partie creuse (15) dudit raccord femelle.

4. Ensemble de raccord d'une conduite de fluide selon la revendication 3, dans lequel ledit deuxième embout femelle (19) dudit raccord femelle (11) comprend un verrouillage de l'embout femelle.

5. Ensemble de raccord d'une conduite de fluide selon la revendication 3 ou 4, dans lequel ledit deuxième embout femelle (52) dudit raccord mâle (12) comprend un verrouillage de l'embout mâle.

6. Ensemble de raccord d'une conduite de fluide selon la revendication 5, dans lequel ledit raccord mâle (12) et ledit raccord femelle (11) sont moulés à partir d'une matière plastique, ladite seconde partie terminale cylindrique (50) dudit raccord mâle étant munie par moulage de dentelures disposées à sa surface extérieure, servant de moyens de prise pour les doigts pour ledit raccord mâle, et ladite paroi essentiellement cylindrique (22) dudit raccord femelle étant munie par moulage de nervures coniques (36) disposées à sa surface extérieure, servant de moyens de prise pour les doigts pour ledit raccord femelle.

7. Ensemble de raccord d'une conduite de fluide selon l'une quelconque des revendications 1 à 6, comprenant en outre une bague frettée (58) en matière plastique destinée à retenir ledit opercule élastique (42) en place sur et autour de ladite première ouverture dudit raccord mâle (12).

8. Ensemble de raccord d'une conduite de fluide selon l'une quelconque des revendications 1 à 7, dans lequel ledit raccord mâle (12) et ledit raccord femelle (11) sont moulés à partir d'une matière plastique.

9. Ensemble de raccord d'une conduite de fluide selon la revendication 8, dans lequel ladite seconde partie terminale cylindrique (50) dudit raccord mâle (12) est munie par moulage de dentelures disposées à sa surface extérieure, servant de moyens de prise pour les doigts pour ledit raccord mâle; et ladite première paroi essentiellement cylindrique (22) dudit raccord femelle (11) est munie par moulage de nervures coniques (36) disposées à sa surface extérieure, servant de moyens de prise pour les doigts pour ledit raccord femelle.

10. Ensemble de raccord d'une conduite de fluide selon la revendication 8 ou 9, dans lequel ladite aiguille creuse (16) comportant ladite première extrémité coupante (24) est réalisée en matière plastique et est formée simultanément avec ledit raccord femelle (11) au cours du processus de moulage.
